(19) Europäisches Patentamt
European Patent Office
Office européen des brevets

(11) EP 0 939 611 B1

# (12) EUROPÄISCHE PATENTSCHRIFT

(45) Veröffentlichungstag und Bekanntmachung des Hinweises auf die Patenterteilung:
**17.10.2001 Patentblatt 2001/42**

(21) Anmeldenummer: **97953875.8**

(22) Anmeldetag: **19.12.1997**

(51) Int Cl.$^7$: **A61K 7/06**

(86) Internationale Anmeldenummer:
**PCT/EP97/07168**

(87) Internationale Veröffentlichungsnummer:
**WO 98/31328 (23.07.1998 Gazette 1998/29)**

(54) **WÄSSRIGE ZUBEREITUNGEN UND IHRE VERWENDUNG**

AQUEOUS COMPOSITIONS AND THEIR USE

COMPOSITIONS AQUEUSES ET LEUR UTILISATION

(84) Benannte Vertragsstaaten:
**AT BE CH DE ES FI FR GB IT LI NL SE**

(30) Priorität: **14.01.1997 DE 19701018**

(43) Veröffentlichungstag der Anmeldung:
**08.09.1999 Patentblatt 1999/36**

(73) Patentinhaber: **BASF AKTIENGESELLSCHAFT
67056 Ludwigshafen (DE)**

(72) Erfinder:
- **HÖSSEL, Peter
D-67105 Schifferstadt (DE)**
- **SPERLING, Karin
D-67433 Neustadt (DE)**
- **SCHEHLMANN, Volker
D-67354 Römerberg (DE)**

(56) Entgegenhaltungen:
**EP-A- 0 256 691**
**EP-A- 0 455 081**
**EP-A- 0 715 843**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft wäßrige Zubereitungen sowie ihre Verwendung in kosmetischen Formulierungen.

Von Lang et al. werden in WO 94/08554 Mittel zur Festigung der Haare, enthaltend mindestens ein Polymer sowie ethoxylierte $C_{12}$-bis $C_{20}$-Fettalkohole und mindestens ein wasserlösliches halogenfreies organisches Lösungsmittel beschrieben. Diese Lösungsmittel trocknen die Haare in unerwünschter Weise aus und führen so zur Haarversprödung. Außerdem können sie zu Hautreizungen führen.

EP-B 155 400 beschreibt Mittel zur Festigung der Frisur und Pflege des Haares, die neben einem quaternisierten Copolymeren aus Vinylpyrrolidon und Dimethylaminoethylmethacrylat, Tetraoxiethylenlaurylether enthalten.

EP-B 331 930 beschreibt ebenfalls Mittel zur Festigung der Frisur und Pflege des Haares mit einem Copolymerisat aus Vinylpyrrolidon und Vinylimidazolmethochlorid sowie Tetraoxiethylenlaurylether.

Nachteil der oben beschriebenen Mittel ist jedoch, daß sie speziell bei hoher Luftfeuchtigkeit zu einem unnatürlichen Verkleben der Haare führen können. Außerdem zeigen diese Mittel noch Schwächen bei der Festigung der Haare. Ein weiterer Nachteil der in EP-B 155 400 genannten Copolymerisate ist die vergleichsweise schlechte Trokkenkämmbarkeit des behandelten Haares.

In EP-A 715843 werden Aerosolschäume mit Copolymeren auf Basis von Vinylcaprolactam, Vinylpyrrolidon und Vinylimidazol, einem Emulgator wie Cetyldimethyl-2-hydroxyethylammoniumdihydrogenphosphat oder Ceteareth-25® (= Polyoxyethlencetylether) und üblichen kosmetischen Hilfsstoffen sowie gegebenenfalls einem Treibmittel beschrieben. Die mit diesen Aerosolschäumen erzielte Festigungswirkung an Haaren ist jedoch noch ungenügend.

Kosmetische Zubereitungen und besonders Zubereitungen für die Haarkosmetik sollten eine Reihe von vorteilhaften Eigenschaften aufweisen. Wichtige Anforderungen an derartige kosmetische Mittel sind beispielsweise

1. Hautverträglichkeit (keine reizenden und toxischen Wirkungen auf der Haut),
2. gutes Haut- und Haargefühl und gute Haftung auf der Haut bzw. auf dem Haar,
3. Wasserfestigkeit,
4. gute Verträglichkeit mit anderen kosmetischen Substanzen,
5. flexible Festigung der Haare (auch bei hoher Luftfeuchtigkeit keine Verklebung der Haare),
6. Verhinderung elektrostatischer Aufladungen der Haare,
7. Vermittlung eines guten Haargefühls (guter Griff, gutes Haarvolumen sowie geringe Klebrigkeit),
8. gute Nasskämmbarkeit,
9. gute Biegefestigkeit
10. Verbesserung des Glanzes
11. gute Löslichkeit in kosmetischen Lösungen und Zubereitungen.

Aufgabe der vorliegenden Erfindung war eine Zubereitung, die möglichst eine Vielzahl der vorteilhaften Eigenschaften erfüllt und die Nachteile der bisher bekannten Mittel nicht aufweist. Die Aufgabe wurde durch die erfindungsgemäße wässrige Zubereitung, enthaltend

a) 0,1 bis 10 Gew.-% eines Copolymerisats auf Basis von N-Vinylcaprolactam, N-Vinylpyrrolidon und N-Vinylimidazol und

b) 0,1 bis 10 Gew.-% mindestens eines Polyoxyethylen-$C_6$-$C_{15}$-monoalkylethers,

gelöst.

Als Copolymerisate (a) eignen sich vorteilhafterweise alle Copolymere, die N-Vinylcaprolactam, N-Vinylpyrrolidon und N-Vinylimidazol enthalten. Bevorzugt eignen sich Copolymere, die erhältlich sind durch radikalisch initiierte Copolymerisation von Monomerengemischen aus

($a_1$) 20 bis 80 Gew. -%, vorzugsweise 40 bis 60 Gew.-% N-Vinylcaprolactam,

($b_1$) 10 bis 60 Gew.-%, vorzugsweise 20 bis 50 Gew.-% N-Vinylpyrrolidon,

($c_1$) 5 bis 50 Gew.-%, vorzugsweise 7 bis 20 Gew.-% eines N-Vinylimidazols oder quaternierten N-Vinylimidazols und

($d_1$) 0 bis 30 Gew.-%, vorzugsweise 0 bis 50 Gew.-% eines weiteren radikalisch copolymerisierbaren Monomeren, das als Homopolymerisat eine Glastemperatur von mehr als 20°C aufweist

und sofern als Monomer ($c_1$) ein nichtquaternisiertes N-Vinylimidazol eingesetzt wird, anschließend vorteilhafterweise eine Quaternierung des Polymeren erfolgt.

Geeignete N-Vinylimidazole (Monomere ($c_1$) sind 1-Vinylimidazol-Derivate der allgemeinen Formel I,

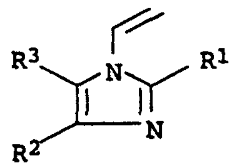

(I)

worin $R^1$ für Wasserstoff, $C_1$-$C_4$-Alkyl- oder Phenyl steht, $R^2$ und $R^3$ gleich oder verschieden sein können und für Wasserstoff oder $C_1$-$C_4$-Alkyl stehen.

Die Vinylimidazole können als freie Basen oder in quaternierter Form eingesetzt werden, wobei die Copolymerisation von quaternierten Vinylimidazolen bevorzugt ist. Setzt man die Vinylimidazole bei der Copolymerisation in Form der freien Basen ein, so erfolgt vorteilhafterweise die Quaternierung nach der Polymerisation.

Zur Quaternisierung des Vinylimidazols eignen sich beispielsweise Alkylhalogenide mit 1 bis 22 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der Vinylimidazole kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.
Ganz besonders bevorzugte Monomere der Gruppe ($c_1$) sind 3-Methyl-1-vinylimidazolium-chlorid und -methylsulfat. Die Quaternierung der Monomeren oder eines Polymeren mit einem der genannten Quaternierungsmittel kann nach allgemein bekannten Methoden erfolgen.

Als Monomere ($d_1$) eignen sich beispielsweise $C_1$-$C_{12}$-Alkylester der Acrylsäure oder der Methacrylsäure wie tert.-Butylacrylat, Isobutylmethacrylat, n-Butylmethacrylat, Methylmethacrylat, Ethylmethacrylat, t-Butylmethacrylat, Isobornylacrylat oder Isobornylmethacrylat oder Acrylamide wie N-tert.-Butylacrylamid oder N-tert.-Octylarylamid. Geeignet sind ferner Monomere, die in Wasser bei 25°C zu mehr als 5 Gew.-% löslich sind, zum Beispiel Acrylsäure, Methacrylsäure, Crotonsäure, N-Methylolmethacrylamid, N-Vinyl-N-methyl-acetamid, N-Vinylformamid, Acrylamid, N, N-Dimethylacrylamid, Methacrylamid, N-Vinyloxazolidon, N-Vinyltriazol, Hydroxyalkyl(meth)acrylate oder Alkylethylenglycol (meth)acrylate mit 1 - 50 Ethylenglycoleinheiten im Molekül.

Ganz besonders bevorzugt sind Copolymere aus

(a) 5 bis 30 Gew.-% 3-Methyl-1-vinyl-imidazoliummethylsulfat,

(b) 40 bis 60 Gew.-% N-Vinylcaprolactam, und

(c) 30 bis 50 Gew.-% N-Vinylpyrrolidon.

Die Herstellung der Polymerisate kann nach dem an sich bekannten Verfahren der radikalisch initiierten Polymerisation erfolgen. Vorzugsweise erfolgt die Herstellung als Lösungspolymerisation in Lösungsmitteln wie Wasser, Methanol, Ethanol, Isopropanol oder Mischungen dieser Lösungsmittel. Man wählt die Mengen an Monomeren und Lösungsmitteln zweckmäßigerweise so, daß 15 bis 60 gew.-%ige Lösungen entstehen. Die Polymerisation erfolgt üblicherweise bei Temperaturen von 60°C bis 130°C und bei Normaldruck oder unter Eigendruck.

Als Initiatoren für die radikalische Polymerisation können die hierfür üblichen Peroxo- und/oder Azo-Verbindungen eingesetzt werden, beispielsweise Dibenzoylperoxid, tert.-Butylperpivalat, tert.-Butyl-2-ethyl-hexanoat, Di-tert.-butylperoxid, tert.-Butylhydroperoxid, Azo-bis-isobutyronitril, Azo-bis-(2-amidinopropan) dihydrochlorid oder 2,2'-Azo-bis-(2-methylbutyronitril). Geeignet sind ebenfalls Initiatormischungen oder übliche Redoxinitiatoren. Die Initiatoren können in üblichen Mengen eingesetzt werden, beispielsweise 0,05 bis 5 Gew.-%, bezogen auf die Menge der zu polymerisierenden Monomere.

Das Molekulargewicht kann gewünschtenfalls durch den Zusatz von Reglern, beispielsweise Verbindungen, die Schwefel in gebundener Form enthalten, eingestellt werden.

Die K-Werte der Polymerisate sollen im Bereich von 10 bis 350, vorzugsweise 50 bis 300 liegen. Der jeweils gewünschte K-Wert läßt sich in an sich bekannter Weise durch die Wahl der Polymerisationsbedingungen, beispielsweise der Polymerisationsdauer und der Initiatorkonzentration und der Reglerkonzentration einstellen. Die K-Werte werden nach Fikentscher, Cellulosechemie, Bd. 13, S. 58-64 (1932) bei 25°C in 0,1 gew.-%iger wäßriger Lösung gemessen.

Im wässrigen kosmetischen Mittel werden die Copolymerisate in einer Menge von 0,1 bis 10 Gew.-%, bevorzugt von 0,1 bis 5 Gew.-%, ganz besonders bevorzugt von 0,5 bis 2,5 Gew.-% verwendet.

Unter ethoxylierten Alkylethern (= Polyoxyethylen-$C_6$-$C_{15}$-monoalkylether) sind Verbindungen der allgemeinen Formel

$$CH_3(CH_2)_m(OCH_2CH_2)_nOH \qquad (II)$$

zu verstehen, wobei die Variablen m 5 bis 14, bevorzugt 7 bis 11 besonders bevorzugt 9 bis 11, und n 1 bis 30 bedeuten, wie beispielsweise Polyoxyethylencapronylether, Polyoxyethylencaprylether, Polyoxyethylenpelargonylether, Polyoxyethylencaprinylether, Polyoxyethylenlaurylether oder Polyoxyethylenmyristylether. Diese Verbindungen sind unter verschiedenen Markennamen beispielsweise den Brij®-Marken erhältlich oder sind durch Ethoxilierung von Fettalkoholen wie 1-Hexanol (= Capronalkohol), 1-Heptanol (= Önanthalkohol), 1-Octanol (= Caprylalkohol), 1-Nonanol (= Pelargonalkohol), 1-Decanol (= Carprinalkohol), 1-Undecanol, 1-Dodecanol (= Laurylalkohol), 1-Tridecanol, 1-Tetradecanol (= Myristylalkohol) oder 1-Pentadecanol synthetisierbar. Dabei kann der Ethoxlierungsgrad der verschiedenen Alkylether stark variieren. Bevorzugt werden Ethoxylierungsgrade (n) von bis 1 bis 25, besonders bevorzugt von 1 bis 20, ganz besonders bevorzugt von 1 bis 10. Auch verzweigtkettige ethoxilierte Alkylether wie Isolaureth-3, Isolaureth-6 oder Isolaureth-10 (zur Herstellung dieser Alkylether wurden verzweigtkettige $C_{12}$-Alkohole verwendet) sind geeignet.

Vorteilhafterweise werden Polyoxyethylencapronylether, Polyoxyethylencaprylether, Polyoxyethylenpelargonylether, Polyoxyethylencaprinylether, Polyoxyethylenlaurylether oder Polyoxyethylenmyristylether verwendet. Bevorzugt sind Polyoxyethylencaprylether, Polyoxyethylenpelargonylether, Polyoxyethylenlaurylether oder Polyoxyethylenmyristylether. Besonders bevorzugt werden die Polyoxyethylenlaurylether und ganz besonders bevorzugt polyoxyethylen(4)laurylether, der beispielsweise unter dem Markennamen Brij® 30 oder Laureth-4® vertrieben wird, Polyoxyethylen(3)laurylether (= Laureth-3) oder Isolaureth-6.

Es werden vorzugsweise Alkylether mit einem HLB-Wert (= Hydrophilie-Lipopihilie-Balance-Wert) von 1 bis 20 vorzugsweise von 10 bis 20 verwendet.

Die ethoxylierten Alkylether höherer Fettalkohole wie beispielsweise Cetylalkohol, Stearylalkohol, Oleylalkohol oder Linolylalkohol liefern deutlich schlechtere Biegefestigkeiten der Haare nach Behandlung als die oben genannten kürzerkettigen Fettalkohole.

Die ethoxylierten Alkylether werden dem wässrigen kosmetischen Mittel vorteilhafterweise in einer Konzentration von 0,1 bis 10 Gew.-%, besonders bevorzugt von 0,1 bis 5 Gew.-% und ganz besonders bevorzugt einer Konzentration von 0,1 bis 1 Gew.-% zugegeben.

Die erfindungsgemäßen wässrigen kosmetischen Mittel können darüber hinaus vorteilhafterweise mindestens ein weiteres filmbildendes Polymer enthalten. Geeignete Polymere sind beispielsweise übliche filmbildende natürliche oder synthetische Polymere.

Unter synthetischen Polymeren sind beispielsweise Homo- oder Copolymere der Acrylsäure oder Methacrylsäure, Copolymerisate aus Acrylsäure und/oder Methacrylsäure und Acrylamiden, Copolymere der Acryl- und/oder Methacrylsäure mit Alkyl(methacrylaten), Copolymere auf Basis von Alkylvinylethern und Maleinsäuremonoalkylestern und/oder Ester anderer Carbonsäuren wie Itaconsäure oder Fumarsäure, Copolymerisate aus Octylacrylamid, Acrylat und Butylaminoethylmethacrylat, sowie Vinylpyrrolidonhomo- oder copolymere, Homo- oder Copolymerisate von N-Vinylcarbonsäureamiden mit weiteren Vinylmonomeren, Vinylpyrrolidonvinylacetatcopolymere oder Copolymerisate aus Vinylpyrrolidon, Vinylacetat, Vinylpropionat und/oder weiteren Vinylmonomeren. Auch Mischungen der genannten Polymere sind geeignet.

Unter natürlichen Polymeren sind beispielsweise Gelatine, Pektine, Galaktomanane, Schellack, Alginate, Chitosane, Cellulose oder deren Derivate zu verstehen. Auch Mischungen der natürlichen Polymere untereinander oder Mischungen mit den synthetischen Polymeren sind geeignet.

Das erfindungsgemäße wässrige kosmetische Mittel enthält die obengenannten filmbildenden Polymere, gegebenenfalls in einer Menge von 0,1 bis 10 Gew.-%, bevorzugt von 0,5 bis 5 Gew.-%.

Darüberhinaus kann das erfindungsgemäße Mittel ein Treibmittel bei Verwendung in Druckbehältern enthalten. Geeignete Treibmittel sind beispielsweise n-Butan, i-Butan, Propan, Difluordichlormethan, Trichlormonofluormethan, Tetrafluordichlorethan, Dimethylether, $N_2$, $N_2O$, $CO_2$ oder Druckluft oder deren Mischungen.

Vorteilhafterweise werden die Treibmittel dem erfindungsgemäßen kosmetischen Mittel in einer Menge bis zu 30 Gew.-%, bevorzugt von 2 bis 10 Gew.-% zugesetzt.

Das erfindungsgemäße Mittel kann aber auch ohne Treibmittel in sogenannten Pumpapplikatoren verwendet werden.

Im erfindungsgemäßen kosmetischen Mittel können organische Lösungsmittel enthalten sein, bevorzugt wasserlösliche organische Lösungsmittel beispielsweise niedere aliphatische Alkohole wie Ethanol, Propanol oder Isopropanol, Glykole wie Ethylenglykol, Propylenglykol oder deren Polyglykole, Polyethylenglykolalkylether von $C_1$- bis $C_4$-Alkoholen, einfache oder gemischte Ketone von $C_1$- bis $C_5$-Alkoholen wie Aceton oder Methylethylketon, bevorzugt Glykole, Polyglykole oder Polyethylenglykolalkylether.

Weiterhin kann das erfindungsgemäße wässrige kosmetische Mittel übliche kosmetische Zusatzstoffe enthalten, wie Farbstoffe, Parfums, Tenside, Eiweißhydrolysate, Rückfetter, Verdicker, Glanzmittel, UV-Absorber, Kräuterextrakte, Konservierungsstoffe wie bakterizide oder fungizide Stoffe, Emulgatoren, wie Sorbitanfettsäureester oder Lanolinderivate, Stabilisatoren wie Magnesiumoder Aluminiumsalze von Fettsäuren, Komplexbildner wie EDTA, Antioxidatien wie BHT, BHA, Ascorbinsäure oder alpha-Tocopherol.

Weitere Bestandteile der erfindungsgemäßen kosmetischen Mittel können kosmetische Wirkstoffe wie Panthenol, Bisabolol, alpha-Tocopherol, alpha-Tocopherolacetat, Aloe Vera, Algenextrakt und/ oder Hyaluronsäure sein.

Die erfindungsgemäßen Polymere eignen sich zur Verwendung als Wirkstoffe in kosmetischen Formulierungen bzw. Zubereitungen, seien es hautkosmetische Zubereitungen wie Flüssigseifen, Körperlotionen, Rasierwasser, Gesichtswasser und anderen kosmetischen Lotionen, vor allem in haarkosmetischen Zubereitungen wie Haarkuren, Haarlotionen, Haarspülungen, Haaremulsionen, Spitzenfluids, Egalisierungsmitteln für Dauerwellen, "Hot-Oil-Treatment"Präparate, Conditioner, Festigerlotionen oder Haarsprays. Je nach Anwendungsgebiet können die erfindungsgemäßen wässrigen kosmetischen Mittel in kosmetischen oder haarkosmetischen Zubereitungen, die als Lösungen, Spülungen, Lotionen, Mousses, Gelen, Aerosolschäumen, Pumpschäumen oder Sprays verwendet werden, enthalten sein.

Beispiele

Formulierung

| | |
|---|---|
| 2.00 g | Polymerwirkstoff |
| 0.20 g | Schaumbildner |
| 0.10 g | Euxyl K 100* |
| 0.40 g ad 100 | Parfümöl Carina / Cremophor RH 40 1:3 Wasser dest. |

* Euxyl K 100 ist ein Konservierungsmittel aus Benzylalkohol, Methylchloroisothiazolinon und Methylisothiazolon

| Nr | Polymer | Schaumbildner (INCI-Bezeichnung) | Festigung subjektive Beurteilung an einer Haarsträhne | Biegefestigkeit (cN) | Klebrigkeit (Note) | Curl Retention (%) |
|---|---|---|---|---|---|---|
| 1 | Luviquat® Hold[1] | Laureth-4® | Note 1 | 390 | 1 | 88 |
| 2 | Luviquat® Hold[1] | HydroxyethylCetyldimoniumphosphat | Note 2 | 170 | 0 | 91 |
| 3 | Luviquat® Hold[1] | Ceteareth-25® | Note 2 | 330 | 1 | 88 |
| 4 | Polyquaternium 11[2] | Laureth-4® | Note 2 | 330 | 2 | 48 |

[1] = Terpolymeres auf Basis 50 Gew.-% N-Vinylcaprolactam , 40 Gew.-% N-Vinylpyrrolidon und 10 Gew.-% N-Vinylimidazoliummethylsulfat

[2] = Copolymer aus N-Vinylpyrrolidon und Dimethylaminoethylmethacrylat quarternisiert mit Diethylsulfat (siehe EP-B 155 400)

Beschreibung der Prüfmethoden:

Vorbehandlung der Haarsträhnen

Haarsträhnen mit einer Länge von 20 cm und einem Gewicht von 2,2 bis 2,6 g wurden in der obengenannten Formulierung getränkt, auf Filterpapier leicht abgedrückt und über Nacht bei 20°C und 65 % rel. Feuchte getrocknet.

Beurteilung der Festigerwirkung an einer Haarsträhne

Die Beurteilung erfolgte subjektiv durch Biegen der Haarsträhnen mit den Fingern einer Hand. Note 1: sehr gute Festigung;
Note 2: gute Festigung; Note 3: schwache Festigung.

Biegefestigkeit

Die Haarsträhne wurde symmetrisch auf zwei zylindrische Rollen (Durchmesser 6 mm, Abstand der beiden Rollen 9 cm) gelegt. In der Mitte der beiden Auflagen wurde mit einer Zug-Druck-Prüfmaschine eine steigende Kraft auf die Strähne ausgeübt. Die Maximalkraft vor dem Durchknicken der behandelten Haarsträhne wird in Centi-Newton (cN) angegeben. Sie ist ein Maß für die Festigungswirkung von Polymeren an Haaren. Jede Polymerlösung wurde an 10 verschiedenen Haarsträhnen geprüft.

Bestimmung der Klebrigkeit

Die zu prüfenden Lösungen (siehe Tabelle) wurden mit einem Rakel mit 120 µm Spaltbreite auf eine Glasplatte aufgebracht. Der Naßfilm wurde bei 75 % relativer Feuchte und 20°C über Nacht in einem Klimaschrank gelagert.
Auf die mit den Lösungen beschichtete Glasplatte wurde ein Plastic-Carbon-Band (Pelikan 2060, 50 mm breit) gelegt. Mit einem Gummistempel der Shore A-Härte 60 + 5 wurde mit 250 N für 10 s belastet. Die Prüfung wurde im Klimaschrank bei 75 % rel. Feuchte durchgeführt.
In dem Maß, in dem die Lösungoberfläche klebrig ist, bleibt die Druckfarbe des Carbon-Bandes auf dem Polymerfilm haften (Beurteilung: Noten von 0 = nicht klebrig bis 5 = sehr stark klebrig, >5: Polymerfilm wird von der Glasplatte abgerissen).

Curl Retention

Für die Bestimmung der Curl Retention wurden Haarsträhnen von 2 g Gewicht und 15,5 cm Länge und aus mittelbraunem europäischen Menschenhaar verwendet.

Bestimmungsmethode

Die Haarsträhnen wurden 1 Stunde in einer Ethanol/Wasser-Lösung (1:1) aufbewahrt, mit Wasser ausgespühlt und dann zweimal mit einer wäßrigen Texapon NSO-Lösung (ca. 0,5 % WS) gewaschen. Anschließend wurden die Haarsträhnen mit Wasser bei ca. 40°C ausgespült bis keine Seifenbildung mehr erkennbar war, gekämmt und in Wasser aufbewahrt.
Die feuchten Haare wurden nun dreimal in die oben beschriebenen Lösungen (siehe oben) eingetaucht, zwischendurch mit den Fingern abgestreift und zwischen Filterpapier ausgedrückt. Danach wurde das Haar um einen Teflonstab (12 mm Durchmesser) gewickelt und mit Filterpapier und Gummiring befestigt. Anschließend wurden die Haarsträhnen 90 min bei 70°C im Wärmeschrank getrocknet.Nach Abkühlen auf Raumtemperatur wurden die Locken abgestreift an einem eigens hierfür angefertigtem Plexiglasgestell aufgehängt und die Lockenlänge (L0) an der angebrachten Skala gemessen.
Für die Bestimmung eines Curl Retention Wertes wurden 10 Haarlokken verwendet. Die Locken wurden in eine Klimakammer mit 20°C und 75 % rel. Luftfeuchte gegeben. Ihre Längen (Lt) wurden nach 5 Stunden gemessen.
Die Curl Retention errechnet sich wie folgt:

$$\text{Curl Retention in \%} = \frac{L-Lt}{L-L0} * 100$$

L = Länge der Haare (15,5 cm)

L0 = Länge der Haarlocke nach dem Trocknen
Lt = Länge der Haarlocke nach Klimabehandlung

Als Curl Retention wurde der Mittelwert aus den 10 Einzelmessungen nach 5 h bei 20°C und 75 % rel. Feuchte angegeben.

## Patentansprüche

**1.** Wäßrige Zubereitung, enthaltend

a) 0,1 bis 10 Gew.-% eines Copolymerisats auf Basis von N-Vinylcaprolactam, N-Vinylpyrrolidon und N-Vinylimidazol und

b) 0,1 bis 10 Gew.-% mindestens eines Polyoxyethylen-$C_6$-$C_{15}$-monoalkylethers.

**2.** Wäßrige Zubereitung nach Anspruch 1, **dadurch gekennzeichnet, daß** es 0,1 bis 5,0 Gew.-% des Copolymerisats auf Basis von N-Vinylcaprolactam, N-Vinylpyrrolidon und N-Vinylimidazol enthält.

**3.** Wäßrige Zubereitung nach Anspruch 1, **dadurch gekennzeichnet, daß** es 0,1 bis 5,0 Gew.-% mindestens eines Polyoxyethylen-$C_6$-$C_{15}$-monoalkylethers enthält.

**4.** Wäßrige Zubereitung nach Anspruch 1 oder 3, **dadurch gekennzeichnet, daß** es mindestens einen Polyoxyethylen-$C_6$-$C_{12}$-monoalkylether enthält.

**5.** Wäßrige Zubereitung nach Anspruch 4, **dadurch gekennzeichnet, daß** es als Polyoxyethylenmonoalkylether Polyoxyethylenlaurylether enthält.

**6.** Wäßrige Zubereitung nach den Ansprüchen 1 bis 5, **dadurch gekennzeichnet, daß** es mindestens ein weiteres filmbildendes Polymer enthält.

**7.** Wäßrige Zubereitung nach den Ansprüchen 1 bis 6, **dadurch gekennzeichnet, daß** es bis zu 30 Gew.-% eines Treibmittels ausgewählt aus der Gruppe n-Butan, i-Butan, Propan, Difluordichlormethan, Trichlormonofluormethan, Tetrafluordichlorethan, Dimethylether, $N_2$, $N_2O$, $CO_2$ oder Druckluft oder deren Mischungen enthält.

**8.** Verwendung eines Mittels gemäß den Ansprüchen 1 bis 7 in kosmetischen Formulierungen.

**9.** Verwendung nach Anspruch 8 in haarkosmetischen Formulierungen.

**10.** Verwendung nach Anspruch 8 oder 9 in Lösungen, Spülungen, Lotionen, Mousses, Gelen, Aerosolschäumen, Pumpschäumen oder Sprays.

## Claims

**1.** An aqueous preparation comprising

a) from 0.1 to 10% by weight of a copolymer based on N-vinylcaprolactam, N-vinylpyrrolidone and N-vinylimidazole and

b) from 0.1 to 10% by weight of at least one polyoxyethylene $C_6$-$C_{15}$-monoalkyl ether.

**2.** An aqueous preparation as claimed in claim 1, which comprises from 0.1 to 5.0% by weight of the copolymer based on N-vinylcaprolactam, N-vinylpyrrolidone and N-vinylimidazole.

**3.** An aqueous preparation as claimed in claim 1, which comprises from 0.1 to 5.0% by weight of at least one polyoxyethylene $C_6$-$C_{15}$-monoalkyl ether.

**4.** An aqueous preparation as claimed in claim 1 or 3, which comprises at least one polyoxyethylene $C_6$-$C_{12}$-monoalkyl ether.

**5.** An aqueous preparation as claimed in claim 4, which comprises polyoxyethylene lauryl ether as polyoxyethylene monoalkyl ether.

**6.** An aqueous preparation as claimed in any of claims 1 to 5, which comprises at least one further film-forming polymer.

**7.** An aqueous preparation as claimed in any of claims 1 to 6, which comprises up to 30% by weight of a propellant selected from the group consisting of n-butane, isobutane, propane, difluorodichloromethane, trichloromonofluoromethane, tetrafluorodichloroethane, dimethyl ether, $N_2$, $N_2O$, $CO_2$ and compressed air or a mixture thereof.

**8.** The use of a composition as claimed in any of claims 1 to 7 in cosmetic formulations.

**9.** The use as claimed in claim 8 in cosmetic hair formulations.

**10.** The use as claimed in claim 8 or 9 in solutions, rinses, lotions, mousses, gels, aerosol foams, pump foams, or sprays.

## Revendications

**1.** Préparation aqueuse, contenant

a) 0,1à 10 % en poids d'un copolymère à base de N-vinycaprolactame, N-vinylpyrrolidone et N-vinylimidazole et
b) 0,1 à 10 % en poids d'au moins un éther de polyoxyéthylène-monoalkyle en $C_6$-$C_{15}$.

**2.** Préparation aqueuse selon la revendication 1,
**caractérisée par le fait qu'**elle contient 0,1 à 5,0 % en poids du copolymère à base de N-vinycaprolactame, N-vinylpyrrolidone et N-vinylimidazole.

**3.** Préparation aqueuse selon la revendication 1,
**caractérisée par le fait qu'**elle contient 0,1 à 5,0 % en poids d'au moins un éther de polyoxyéthylène-monoalkyle en $C_6$-$C_{15}$.

**4.** Préparation aqueuse selon la revendication 1 ou 3, **caractérisée par le fait qu'**elle contient au moins un éther de polyoxyéthylène-monoalkyle en $C_6$-$C_{12}$.

**5.** Préparation aqueuse selon la revendication 4,
**caractérisée par le fait qu'**elle contient comme éther de polyoxyéthylène-monoalkyle de l'éther de polyoxyéthylènelauryle.

**6.** Préparation aqueuse selon les revendications 1 à 5, **caractérisée par le fait qu'**elle contient au moins un autre polymère filmogène.

**7.** Préparation aqueuse selon les revendications 1 à 6, **caractérisée par le fait qu'**elle contient jusqu'à 30 % en poids d'un agent propulseur choisi dans le groupe du n-butane, iso-butane, propane, difluorodichlorométhane, trichloromonofluorométhane, tétrafluorodichloroéthane, éther diméthylique, $N_2$, $N_2O$, $CO_2$ ou de l'air comprimé ou leurs mélanges.

**8.** Utilisation d'un produit selon les revendications 1 à 7 dans des formulations cosmétiques.

**9.** Utilisation selon la revendication 8 dans des formulations cosmétiques capillaires.

**10.** Utilisation selon la revendication 8 ou 9 dans des solutions, des produits de rinçage, des lotions, des mousses, des gels, des mousses en aérosol, des mousses par pompage ou des sprays.